# EUROPEAN PATENT APPLICATION

(11) **EP 2 238 903 A1**
(43) Date of publication of application: **13.10.2010**
(21) Application number: 08722521.5
(22) Date of filing: 19.03.2008
(51) Int. Cl.: A61B 5/157, A61B 5/1473

(54) **BIOLOGICAL FLUID EXTRACTING CIRCUIT SUBSTRATE**

(30) Priority: 06.02.2008 JP 2008026839
(71) Applicant: NITTO DENKO CORPORATION, Ibaraki-shi Osaka 567-8680 (JP)
(72) Inventor: ISHII, Jun, Ibaraki-shi Osaka 567-8680 (JP); NAITO, Toshiki, Ibaraki-shi Osaka 567-8680 (JP); KANETO, Masayuki, Ibaraki-shi Osaka 567-8680 (JP); OHSAWA, Tetsuya, Ibaraki-shi Osaka 567-8680 (JP)
(74) Representative: HOFFMANN EITLE
(86) International application number: PCT/JP2008/055147
(87) International publication number: WO 2009/098787

(57) **Abstract**

A circuit board for body fluid collection includes a circuit board portion including an insulating layer and a conductor pattern which is supported by the insulating layer and in which an electrode, a terminal to be connected to a device for measuring the component of a body fluid, and a wire which electrically connects the electrode and the terminal are integrally provided. The circuit board for body fluid collection comprises a puncture needle for extracting the body fluid by performing puncturing, which is formed integrally with the circuit board portion and which is protruded from the circuit board portion, and a guard portion for guarding the tip of the puncture needle, which is arranged in opposition to the puncture needle in the lower flow side in the puncture direction of the puncture needle.

## Description

### Technical Field

The present invention relates to a circuit board for body fluid collection, and particularly to a circuit board for body fluid collection which is connected to a device for measuring a component of a body fluid, and used to measure a component of a body fluid.

### Background Art

Diabetes mellitus includes insulin-dependent (type I) diabetes and non-insulin-dependent (type II) diabetes. The former type of diabetes necessitates regular administration of insulin. Therefore, for a patient with the former type of diabetes, a treatment method has been employed in which the patient collects his or her blood, measures his or her blood sugar value, and administers to himself or herself insulin at a dosage in accordance with the blood sugar value.
A blood-sugar-value measuring device which allows a patient to personally collect blood, and measure a blood sugar value has been known solely to such a patient.

For example, there has been proposed a fluid collecting device including a reaction zone which is provided at the center of a main body and into which electrodes are inserted, a puncture needle outwardly protruding from the center of the main body, and a capillary channel providing communication between the electrodes and the puncture needle (see, e.g., Patent Document 1 shown below).
Patent Document 1: Japanese Unexamined Patent No. 2004-493

### Disclosure of the Invention

### Problems to be Solved

In the fluid collecting device described in Patent Document 1, the puncture needle and the reaction zone are formed integrally with the main body so that preparations for measurement are easy. However, in the fluid collecting device, the electrodes which are members separate from the reaction zone are inserted into the reaction zone to measure a blood component. This leads to a problem that the accuracy of sensing blood is unstable, and accurate measurement cannot be performed.
In addition, in the fluid collecting device described in Patent Document 1, the puncture needle outwardly protrudes from the center of the main body, and is exposed therefrom. As a result, the puncture needle is likely to come in contact with another member, and damaged thereby. In such a case, it is difficult to cause breeding with the puncture needle upon puncture.

An object of the present invention is to provide a circuit board for body fluid collection which allows accurate measurement of a component of a body fluid with a simple structure, and can prevent damage to a puncture needle.

### Means for Solving the Problems

To attain the foregoing object, a circuit board for body fluid collection of the present invention includes a circuit board portion including an insulating layer, and a conductive pattern supported on the insulating layer and integrally including an electrode, a terminal for connecting to a device for measuring a component of a body fluid, and a a wire electrically connecting the electrode and the terminal, a puncture needle integrally formed with the circuit board portion, and protruding from the circuit board portion in order to extract the body fluid by puncture, and a guard portion disposed downstream of the puncture needle in a puncture direction in opposing relation thereto in order to guard a tip of the puncture needle.

The circuit board for body fluid collection includes the circuit board portion, and the puncture needle formed integrally therewith. Therefore, it is possible to cause the body fluid to flow out by puncture with the puncture needle, and easily bring the flown-out body fluid into contact with the electrode of the circuit substrate. Additionally, in the circuit board for body fluid collection, the electrode, the terminal, and the a wire are provided integrally as the conductive pattern in the circuit board portion. Therefore, it is possible to improve the accuracy of sensing a component of the body fluid in contact with the electrode, and improve measurement accuracy. As a result, the circuit board for body fluid collection allows accurate measurement of a component of the body fluid with a simple structure, and allows easy operation.

In addition, in the circuit board for body fluid collection, the tip of the puncture needle can be guarded by the guard portion, and therefore damage to the puncture needle can be prevented. As a result, damage to the puncture needle can be reliably prevented prior to puncture. Therefore, it is possible to cause the body fluid to reliably flow out with the tip of the puncture needle, and sense the component of the flown-out body fluid.
It is preferable that the circuit board for body fluid collection of the present invention further includes a releasing portion for releasing the guarding of the tip of the puncture needle by the guard portion, by bringing the guard portion and the puncture needle away from each other.

The releasing portion allows the guard portion and the puncture needle to be brought away from each other, and allows release of the guarding by the guard portion. Therefore, it is possible to prevent damage to the puncture needle prior to puncture, while it is possible to easily release the guarding by the guard portion, and reliably expose the puncture needle at the time of puncture.
It is more preferable that the guard portion is supported by the circuit board portion, and the releasing portion is bendably provided in a portion of the guard portion located upstream of the tip of the puncture needle in the puncture direction.

By bending the releasing portion at the portion of the guard portion located upstream of the tip of the puncture needle in the puncture direction, it is possible to reliably bring the guard portion and the puncture needle away from each other, and reliably release the guarding by the guard portion.
It is also more preferable that the releasing portion is bendably provided in a portion of the puncture needle or the circuit board portion located upstream of the tip of the puncture needle in the puncture direction.

By bending the releasing portion at the portion of the puncture needle or the circuit board portion located upstream of the tip of the puncture needle in the puncture direction, it is possible to reliably bring the guard portion and the puncture needle away from each other, and reliably release the guarding by the guard portion.

### Effect of the Invention

The circuit board for body fluid collection allows accurate measurement of a component of a body fluid with a simple structure, and allows easy operation.

### Brief Description of the Drawings

[FIG. 1] FIG. 1 shows a circuit substrate for blood collection as an embodiment of a circuit board for body fluid collection of the present invention,
   (a) showing a plan view, and
   (b) showing a longitudinal cross-sectional view along the line A-A in (a).
[FIG. 2] FIG. 2 is a production process view showing an example of a producing method of the circuit substrate for blood collection shown in FIG. 1,
   (a) showing the step of preparing a metal board,
   (b) showing the step of forming an insulating base layer,
   (c) showing the step of forming a conductive pattern,
   (d) showing the step of forming an insulating cover layer,
   (e) showing the step of trimming the metal board, and
   (f) showing the step of coating electrodes with a chemical agent.

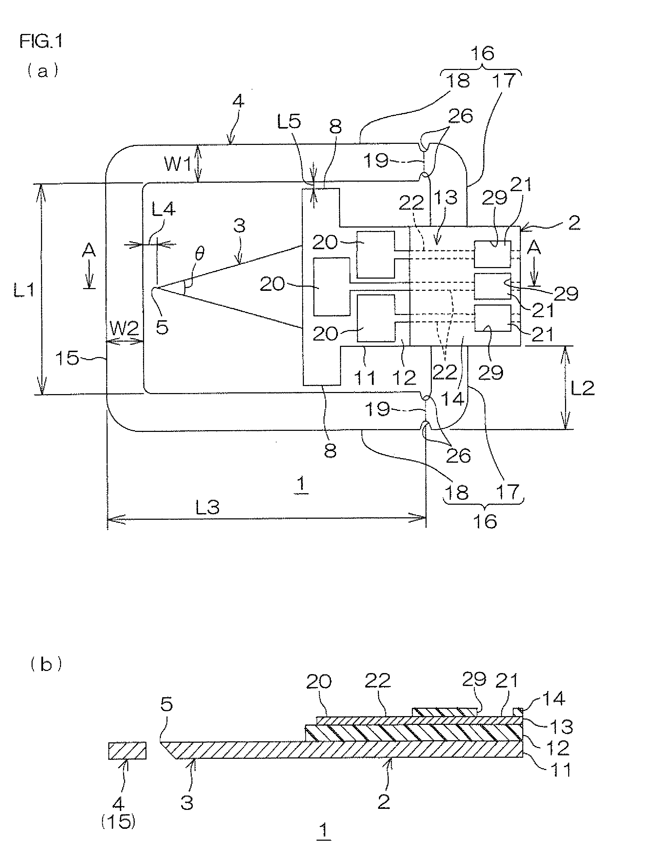

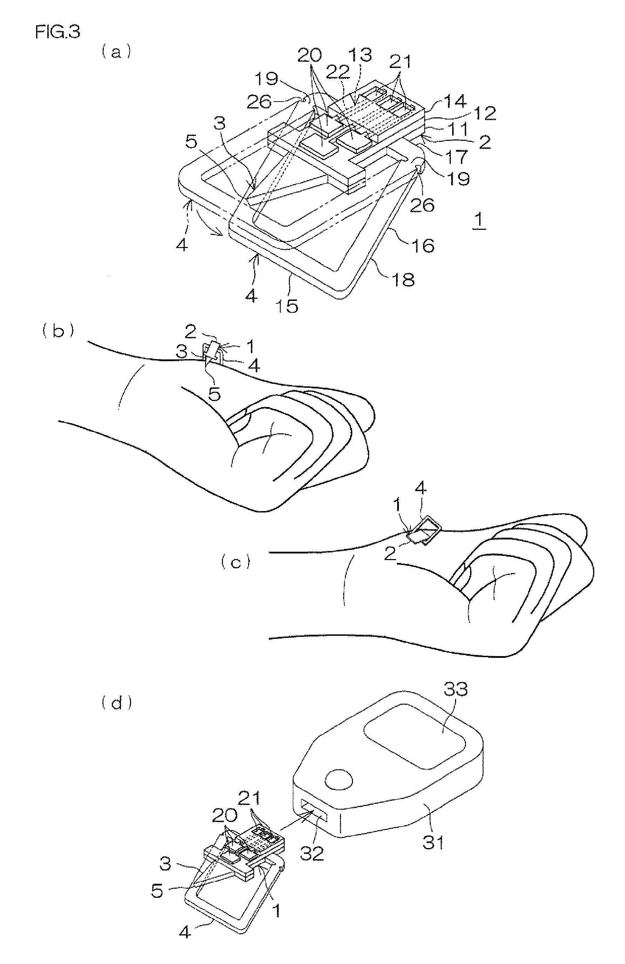

[FIG. 3] FIG. 3 is an illustrative view showing an example of a method of using the circuit substrate for blood collection shown in FIG. 1,
   (a) showing a state where the guarding by a guard portion is released,
   (b) showing a state where a puncture needle punctures,
   (c) showing a state where the electrodes are brought into contact with a punctured portions, and
   (d) showing a state where the circuit substrate for blood collection is inserted into a blood-sugar-value measuring device.

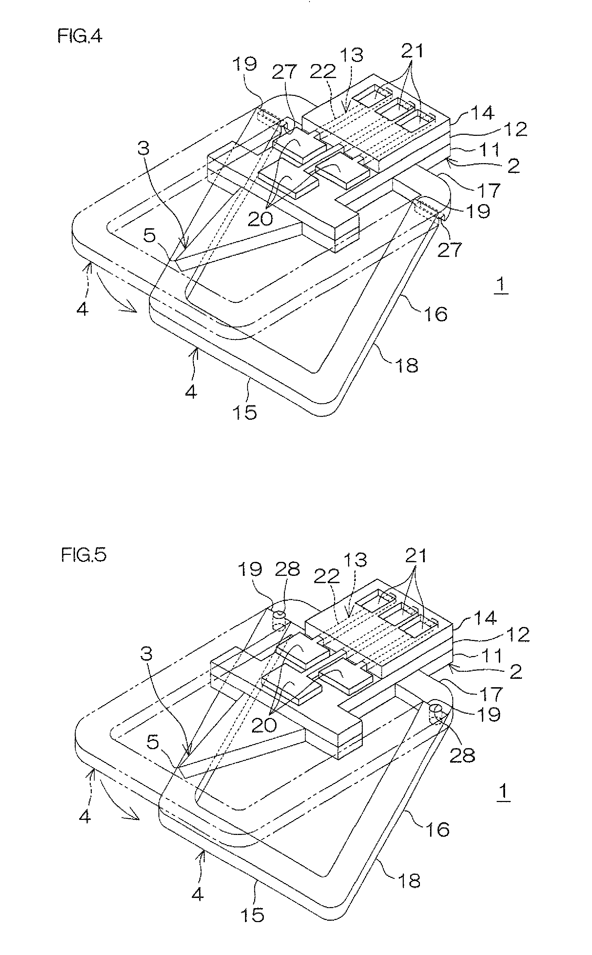

[FIG. 4] FIG. 4 is an illustrative view showing an example of a method of using a circuit substrate for blood collection which is another embodiment (implementation in which guard-side groove portions are formed in bend portions) of the circuit board for body fluid collection of the present invention.
[FIG. 5] FIG. 5 is an illustrative view showing an example of a method of using a circuit substrate for blood collection which is still another embodiment (implementation in which guard-side through holes are formed in bend portions) of the circuit board for body fluid collection of the present invention.

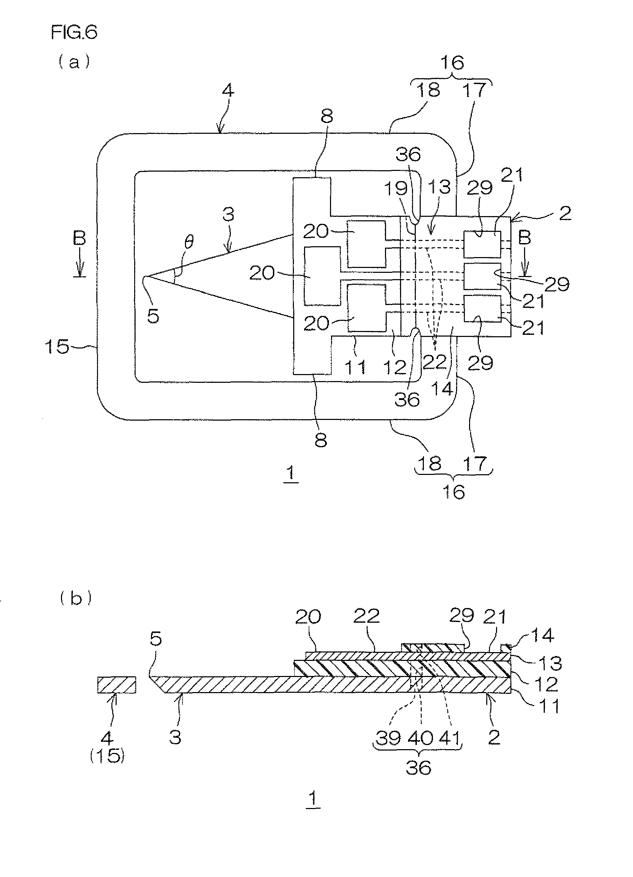

[FIG. 6] FIG. 6 shows a circuit substrate for blood collection which is yet another embodiment (implementation in which a bend portion is provided in a circuit board portion) of the circuit board for body fluid collection of the present invention,
   (a) showing a plan view, and
   (b) showing a longitudinal cross-sectional view along the line B-B in (a).

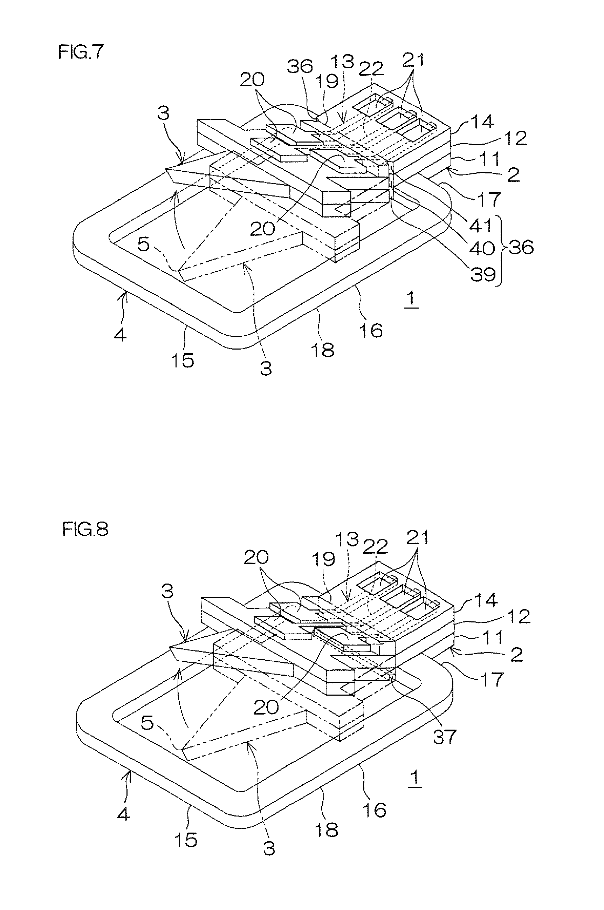

[FIG. 7] FIG. 7 is an illustrative view showing an example (implementation in which circuit-side recessed portions are formed in a bend portion) of a method of using the circuit substrate for blood collection shown in FIG. 6.
[FIG. 8] FIG. 8 is an illustrative view showing an example of a method of using a circuit substrate for blood collection which is still another embodiment (implementation in which a circuit-side groove portion is formed in a bend portion) of the circuit board for body fluid collection of the present invention.

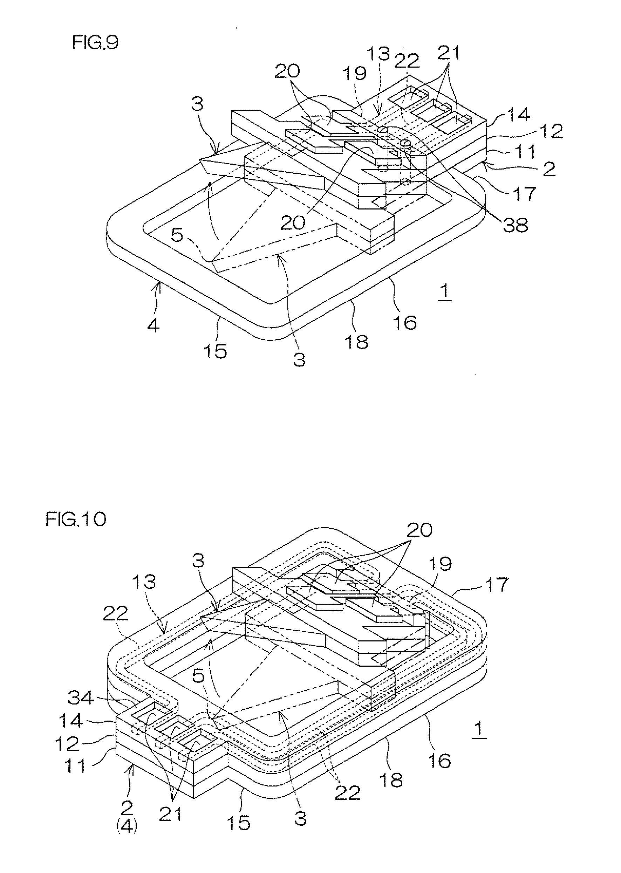

[FIG. 9] FIG. 9 is an illustrative view showing an example of a method of using a circuit substrate for blood collection which is yet another embodiment (implementation in which circuit-side through holes are formed in a bend portion) of the circuit board for body fluid collection of the present invention.
[FIG. 10] FIG. 10 is an illustrative view showing an example of a method of using a circuit substrate for blood collection which is still another embodiment (implementation in which a circuit board portion also serves as a guard portion) of the circuit board for body fluid collection of the present invention.

### Embodiments of the Invention

FIG. 1 shows a circuit substrate for blood collection as an embodiment of a circuit board for body fluid collection of the present invention, (a) showing a plan view, and (b) showing a longitudinal cross-sectional view along the line A-A in (a).
In FIG. 1, the circuit substrate for blood collection 1 is used in conjunction with a blood-sugar-value measuring device 31 (see FIG. 3(d)) for a patient to puncture the skin of his or her finger or the like, collect his or her blood, and measure an amount of glucose in the collected blood. The circuit substrate for blood collection 1 is prepared as a disposable type to be disposed of after each measurement.

As shown in FIG. 1(a), the circuit substrate for blood collection 1 includes a circuit board portion 2, a puncture needle 3, and a guard portion 4.
The circuit board portion 2 is disposed in a longitudinal direction (left-right direction across the surface of the paper with FIG. 1(a)) of the circuit substrate for blood collection 1, i.e., disposed upstream (on the right-hand side of FIG. 1(a)) of the puncture needle 3 described later in a puncture direction. The circuit board portion 2 is formed in a generally rectangular (oblong) plan view shape which is long in the puncture direction.

As shown in FIG. 1(b), the circuit board portion 2 includes a metal board 11, an insulating base layer 12 as an insulating layer laminated on a surface of the metal board 11, a conductive pattern 13 laminated on the surface of the insulating base layer 12, and an insulating cover layer 14 provided on the surface of the insulating base layer 12 so as to cover the conductive pattern 13.
As shown in FIG. 1(a), the metal board 11 is formed of a metal foil or the like in a generally rectangular plan view shape extending in the longitudinal direction in the circuit board portion 2. Examples of a metal used to form the metal board 11 include nickel, chromium, iron, and stainless steel (SUS304, SUS430, or SUS316L). Preferably, stainless steel is used. The thickness of the metal board 11 is in a range of, e.g., 10 to 300 µm, or preferably 20 to 100 µm. When the thickness thereof is less than 10 µm, skin may not be able to be punctured due to an insufficient strength (described later), or a puncture needle 3 (describes later) may not be able to be reliably guarded by the guard portion 4 (described later). On the other hand, when the thickness thereof is in excess of 300 µm, a user may feel pain upon puncture, and skin may be excessively damaged, or bend portions 19 (described later) may not be able to be bent smoothly.

The insulating base layer 12 is formed in the same shape as that of the metal board 11 when viewed in plan view in the circuit board portion 2. Examples of an insulating material used to form the insulating base layer 12 include synthetic resins such as a polyimide resin, a polycarbonate resin, a polyethylene resin, a polyethylene terephthalate resin, an epoxy resin, and a fluorine resin. In terms of mechanical endurance and resistance to a chemical agent, a polyimide resin is preferably used. The thickness of the insulating base layer 12 is in a range of, e.g., 3 to 50 µm, or preferably 5 to 25 µm. When the thickness thereof is less than 3 µm, an insulation defect such as a pinhole may occur. On the other hand, when the thickness thereof is in excess of 50 µm, cutting and trimming may be hard to perform.

The conductive pattern 13 is supported on the insulating base layer 12 in the circuit board portion 2, and includes three electrodes 20, three terminals 21, and three wires 22.
The three electrodes 20 are disposed on the downstream-side portion of the circuit board portion 2 in the puncture direction. These electrodes 20 are each formed in a generally rectangular plan view shape, of which the two are arranged in parallel in a widthwise direction (direction perpendicular to the longitudinal direction), and the remaining one is disposed downstream of the foregoing two in the puncture direction. Each of the three electrodes 20 corresponds to any of a working electrode, a counter electrode, and a reference electrode. The length of one side of each of the electrodes 20 is in a range of, e.g., 100 µm to 2.5 mm. The three electrodes 20 are each disposed within a range of, e.g., 0.2 to 5 mm, or preferably 0.5 to 3 mm from the tip 5 of the puncture needle 3 in the puncture direction. When the distances between the tip 5 of the puncture needle 3 and the electrodes 20 are excessively short, the electrodes 20 may pierce skin together with the puncture needle 3, and a chemical agent 30 (described later) coated on the surfaces of the electrodes 20 may be dispersed into a body to inhibit precise measurement. On the other hand, when the distances between the tip 5 of the puncture needle 3 and the electrodes 20 are excessively long, a structure for using suction or capillarity is needed to introduce blood from the puncture needle 3 into the electrodes 20.

The three terminals 21 are provided correspondingly to the three electrodes 20, and disposed on the upstream-side portion of the circuit board portion 2 in the puncture direction so as to be connected to the blood-sugar-value measuring device 31. These terminals 21 are each formed in a generally rectangular plan view shape slightly smaller than each of the electrodes 20. The three terminals 21 are also arranged in parallel in the widthwise direction.
The three wires 22 are arranged in parallel to be spaced apart from each other in the widthwise direction. The three wires 22 are provided along the longitudinal direction to electrically connect the individual electrodes 20 and the individual terminals 21 corresponding thereto. Each of the electrodes 20, each of the terminals 21, and the wire 22 connected to the electrode 20 and the terminal 21 are continuously and integrally provided. The widthwise length of each of the wires 22 is in a range of, e.g., 0.01 to 2 mm. The longitudinal length of each of the wires 22 is in a range of, e.g., 5 to 28 mm.

Examples of a conductive material used to form the conductive pattern 13 include metals such as iron, nickel, chromium, copper, gold, silver, platinum, and an alloy thereof. A conductive material is selected appropriately in terms of adhesion to the insulating base layer 12 and the insulating cover layer 14 and easy processing. It is also possible to laminate two or more kinds of conductive materials. The thickness of the conductive pattern 13 is in a range of, e.g., 5 to 50 µm, or preferably 10 to 20 µm.

The insulating cover layer 14 is provided on the surface of the insulating base layer 12 exposed from the conductive pattern 13 so as to cover each of the wires 22 in the circuit board portion 2. Specifically, the downstream end edge of the insulating cover layer 14 in the puncture direction is formed in a linear shape along the widthwise direction on the side upstream of the three electrodes 20 in the puncture direction so as to expose the three electrodes 20. The insulating cover layer 14 is formed with openings 29 for exposing the respective terminals 21. As an insulating material for forming the insulating cover layer 14, the same insulating material as that of the insulating base layer 12 shown above is used. The thickness of the insulating cover layer 14 is in a range of, e.g., 2 to 50 µm.

The circuit board portion 2 is also provided with stopper portions 8.
The stopper portions 8 are provided at the downstream end portions of the circuit substrate 2 in the puncture direction so as to protrude from the both widthwise outer ends thereof along the both widthwise outsides thereof. The protruding lengths of the stopper portions 8 on both widthwise outsides are in a range of, e.g., 0.1 to 2 mm. Each of the stopper portions 8 is formed in a generally rectangular plan view shape, and the downstream end edge thereof in the puncture direction is formed in the same straight line as the downstream end edge of the insulating base layer 12 along the widthwise direction. The downstream end edge of each of the stopper portions 8 in the puncture direction is disposed to be spaced apart from the tip 5 of the puncture needle 3 toward the side upstream of the puncture needle 3 in the puncture direction by 0.3 to 2.0 mm. When the positions of the stopper portions 8 are within the range shown above, excessive puncture with the puncture needle 3 can be reliably prevented.

The stopper portions 8 are each formed of the metal board 11 and the insulating base layer 12. The metal board 11 and the insulating base layer 12 are formed in the same shape when viewed in plan view in each of the stopper portions 8.
The puncture needle 3 is provided for collecting blood by puncture. That is, the puncture needle 3 is disposed downstream of the circuit board portion 2 in the puncture direction in adjacent relation thereto, and formed integrally with the circuit board portion 2. Specifically, the puncture needle 3 protrudes downstream from the circuit board portion 2 in the puncture direction. The puncture needle 3 is formed in a generally triangular plan view shape (in an isosceles triangular shape) having the tip 5 (downstream end portion in the puncture direction) pointed at an acute angle along the longitudinal direction. The puncture needle 3 is formed from the metal board 11.

The angle θ of the tip 5 of the puncture needle 3 is in a range of, e.g., 10 to 30°, or preferably 15 to 25°. When the angle θ of the tip 5 is less than 10°, skin may not be able to be punctured due to an insufficient strength. On the other hand, when the angle θ thereof is in excess of 30°, skin may be hard to puncture. The longitudinal length of the puncture needle 3 is in a range of, e.g., 0.3 to 2 mm.
The guard portion 4 is provided for guarding the tip 5 of the puncture needle 3. That is, the guard portion 4 is formed in a generally rectangular frame shape surrounding the puncture needle 3 when viewed in plan view. The guard portion 4 integrally includes support portions 16 and a main body portion 15.

The support portions 16 are each supported by the circuit board portion 2, and formed in a generally L-shaped plan view shape continued to the circuit board portion 2. The support portions 16 integrally include respective base portions 17 protruding from the midway portions of the circuit board portion 2 in the puncture direction (from between the upstream-side portion and downstream-side portion thereof in the puncture direction) toward the both widthwise outsides, and respective lateral portions 18 extending downstream in the puncture direction from the both widthwise outer end portions of the base portions 17. The downstream end portions of the lateral portions 18 in the puncture direction are located downstream of the tip 5 of the puncture needle 3 in the puncture direction.

The main body portion 15 is supported by the support portions 16. That is, the main body portion 15 is spanned between the respective downstream end portions of the two lateral portions 18 in the puncture direction, and formed in a generally rectangular plan view shape extending along the widthwise direction. Consequently, the main body portion 15 is located downstream of the tip 5 of the puncture needle 3 in the puncture direction in opposing relation thereto. Specifically, the main body portion 15 is disposed in the same plane as the tip 5 of the puncture needle 3 to be spaced apart therefrom.

The length (widthwise length which is the widthwise distance between the two lateral portions 18) L1 of the main body portion 15 is in a range of, e.g., 1 to 20 mm, or preferably 3 to 10 mm. The length (widthwise length which is the widthwise distance between the widthwise outer end edge of the circuit board portion 2 and the widthwise outer end edge of the lateral portion 18) L2 of the base portion 17 is in a range of, e.g., 0.3 to 10 mm, or preferably 1 to 5 mm. The length (length in the puncture direction which is the distance between the downstream end edge of the base portion 17 in the puncture direction and the downstream end edge of the main body portion 15 in the puncture direction) L3 of the lateral portion 18 is in a range of, e.g., 1 to 20 mm, or preferably 3 to 10 mm. The distance L4 between the main body portion 15 and the puncture needle 3 in the puncture direction is in a range of, e.g., 0.05 to 3 mm, or preferably 0.1 to 2 mm. Further, the widthwise distance L5 between the lateral portion 18 and the stopper portion 8 is in a range of, e.g., 0.1 to 3 mm, or preferably 0.5 to 2 mm.

The width (length of the base portion 17 in the puncture direction or the widthwise length of the lateral portion 18) W1 of each of the support portions 16 of the guard portion 4 and the width (length in the puncture direction) W2 of the main body portion 15 of the guard portion 4 may be the same as or different from each other. The widths W1 and W2 are each in a range of, e.g., 0.5 to 5 mm, or preferably 1 to 3 mm.
The guard portion 4 is formed from the metal board 11.
Also, the guard portion 4 includes bend portions 19 each as a releasing portion.

As shown by the 1-dot broken line of FIG. 1(a) and in FIG. 3(a), the bend portions 19 are provided to release the guarding of the tip 5 of the puncture needle 3 by the guard portion 4 at the time of puncture with the puncture needle 3 described later. That is, the bend portions 19 are provided in the portions of the guard portion 4 located upstream of the tip 5 of the puncture needle 3 in the puncture direction, i.e., at the upstream end portions of the lateral portions 18 in the puncture direction. Specifically, the bend portions 19 are formed as linear portions extending along the widthwise direction between the base portions 17 and the lateral portions 18.

The bend portions 19 are formed as narrower portions (constricted portions) having reduced widths by guard-side recessed portions 26 recessed widthwise inwardly at the adjoining portions where the lateral portions 18 and the base portions 17 adjoin each other.
The guard-side recessed portions 26 are formed in such a manner that the metal board 11 is cut into generally semi-cylindrical shapes over a thickness direction at the both widthwise end portions of the adjoining portions between the lateral portions 18 and the base portions 17. The inner diameters (specifically the maximum lengths in the widthwise direction or the maximum lengths in the puncture direction) of the guard-side recessed portions 26 are in a range of, e.g., 100 to 3000 µm, or preferably 300 to 2000 µm.

Thus, the bend portions 19 are formed as fragile portions between the lateral portions 18 8 and the base portions 17 so that the main body portion 15 and the lateral portions 18 are provided bendably with respect to the base portions 17.

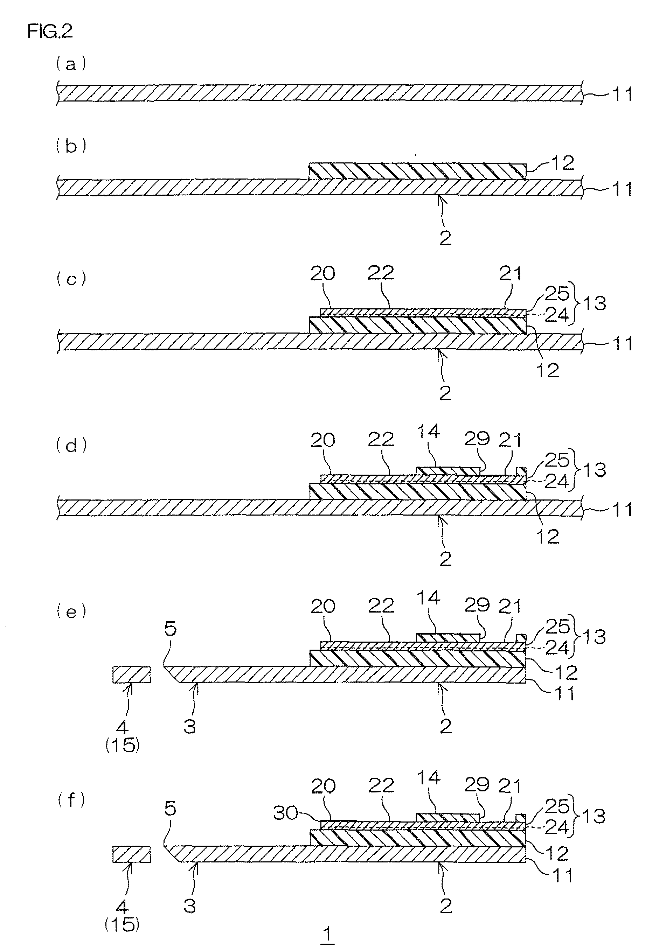

FIG. 2 is a production process view showing an example of a producing method of the circuit substrate for blood collection 1 shown in FIG. 1. Next, referring to FIG. 2, a description is given to the producing method of the circuit substrate for blood collection 1.
First in this method, the metal board 11 is prepared, as shown in FIG. 2(a). The metal board 11 is prepared as, e.g., an elongated metal foil which allows a large number of the metal boards 11 to be ensured. From the elongated metal foil, a plurality of the circuit substrate for blood collection 1 are produced by trimming (described later) the individual metal boards 11.

Next in this method, the insulating base layer 12 is formed on a surface of the metal board 11, as shown in FIG. 2(b). For the formation of the insulating base layer 12, there is used a method in which, e.g., a varnish of a photosensitive synthetic resin is coated on the surface of the metal board 11, photoprocessed, and then cured, a method in which, e.g., a film of a synthetic resin is laminated on the surface of the metal board 11, an etching resin in the same pattern as that of the insulating base layer 12 is laminated on the surface of the film, and then the film exposed from the etching resist is wet-etched, a method in which, e.g., a film of a synthetic resin mechanically punched in advance is laminated on the surface of the metal board 11, a method in which, e.g., a film of a synthetic resin is laminated on the surface of the metal board 11, and then subjected to discharge processing or laser processing, or the like. In terms of processing accuracy, the method in which a varnish of a photosensitive synthetic resin is coated on the surface of the metal board 11, photoprocessed, and then cured is preferably used.

Then, in this method, the conductive pattern 13 is formed, as shown in FIG. 2(c). For the formation of the conductive pattern 13, a known patterning method for forming printed a wire is used, such as an additive method or a subtractive method. In terms of enabling the formation of a minute pattern, the additive method is preferably used. In the additive method, e.g., a metal thin film 24 (broken line) is formed on the surface of the insulating base layer 12 by chemical vapor deposition or sputtering, a plating resist is formed on the surface of the metal thin film 24, and then, a plated layer 25 is formed on the surface of the metal thin film 24 exposed from the plating resist by electrolytic plating using the metal thin film 24 as a seed film.

The conductive pattern 13 can also be formed only from the metal thin film 24 by chemical vapor deposition or sputtering.
In the formation of the conductive pattern 13, it is also possible to further form a plated layer of a different metal on the surface of each of the electrodes 20 and the terminals 21 by electrolytic plating or electroless plating, though not shown. The thickness of the metal plated layer is preferably in a range of 0.05 to 20 µm.

Then, in this method, the insulating cover layer 14 is formed, as shown in FIG. 2(d). For the formation of the insulating cover layer 14, the same method as the method used to form the insulating base layer 12 is used. Preferably, a method is used in which a varnish of a photosensitive synthetic resin is coated on the surface of the insulating base layer 12 so as to cover the conductive pattern 13, and then cured after photoprocessing. Note that, in the case of forming the insulating cover layer 14 in a pattern having the openings 29, the insulating cover layer 14 may be formed appropriately in the pattern having the openings 29. The openings 29 can also be bored by a method in which, e.g., discharge processing is performed, a method in which, e.g., laser processing is performed, or the like.

Thereafter, as shown in FIG. 2(e), the metal board 11 is trimmed to simultaneously form the circuit board portion 2, the puncture needle 3, and the guard portion 4. For the trimming of the metal board 11, there is used, e.g., discharge processing, laser processing, mechanical punching processing (such as, e.g., punching), etching processing, or the like. In terms of easy cleaning after processing, etching processing (wet etching) is preferably used.

In this manner, the circuit substrate for blood collection 1 can be obtained in which the puncture needle 3 and the guard portion 4 are integrally formed with the circuit board portion 2. In other words, the circuit substrate for blood collection 1 can be obtained in which the circuit board portion 2, the puncture needle 3, and the guard portion 4 include the common metal board 11. In the obtained circuit board for blood collection 1, as shown in FIG. 2(f), the chemical agent 30 is coated, i.e., e.g., glucose oxidase, glucose dehydrogenase, or the like as an enzyme and, e.g., potassium ferricyanide, ferrocene, benzoquinone, or the like as a mediator are coated alone or in combination on each of the electrodes 11. For the coating of the chemical agent 30, an appropriate method such as, e.g., a dipping method, a spray method, or an inkjet method is used.

Depending on the type of the chemical agent 30, after a plated layer of a different metal is formed on the surface of each of the electrodes 20 as described above, it is also possible to further form a coating of a different metal in advance, and then provide a predetermined potential difference therebetween. Specifically, it is shown by way of example to form a gold plated layer, and then further coat silver or a silver chloride on the surface of the gold plated layer.
FIG. 3 is an illustrative view showing an example of a method of using the circuit substrate for blood collection 1 shown in FIG. 1. Next, referring to FIG. 3, the method of using the circuit substrate for blood collection 1 is described.

As described above, the circuit substrate for blood collection 1 is used in conjunction with the blood-sugar-value measuring device 31 (FIG. 3(d)) for a patient to puncture the skin of his or her finger or the like, collect his or her blood, and measure an amount of glucose in the collected blood.
To measure the amount of glucose in the blood, the guarding of the tip 5 of the puncture needle 3 by the guard portion 4 is released first, as shown in FIG. 3(a). That is, prior to puncture (specifically, after the production of the circuit substrate for blood collection 1 and prior to puncture with the puncture needle 3), the guard portion 4 is disposed downstream of the tip 5 of the puncture needle 3 in the puncture direction in opposing relation thereto, as shown by the imaginary line of FIG. 3(a) so that the tip 5 of the puncture needle 3 is guarded by the guard portion 4.

To release the guarding by the guard portion 4, as shown by the arrow of FIG. 3(a), the lateral portions 18 and the main body portion 15 are bent along the bend portions 19 with respect to the base portions 17. By the bending, as shown by the solid line of FIG. 3, the guard portion 4 and the puncture needle 3 are brought away from each other in a direction (direction of rotational movement in which the main body portion 15 rotationally moves around the bend portions 19 as an axis) crossing the puncture direction. Specifically, by rotationally moving the guard portion 4 relative to the puncture needle 3 toward the back surface side of the circuit board portion 2 (toward the metal board 11) in the direction of rotational movement, the guard portion 4 is withdrawn downwardly from a location downstream of the puncture needle 3 in the puncture direction.

in this manner, guarding provided by the guard portion 4 against puncture with the puncture needle 3 can be released.
Then, as shown in FIG. 3(b), the patient punctures his or her finger or the like with the puncture needle 3 to extract an extremely small amount of blood therefrom. At this time, when the stopper portions 8 come to abut on skin during puncture with the puncture needle 3, further puncture is restricted thereby.

Immediately thereafter, as shown in FIG. 3(c), the electrodes 20 are brought closer into contact with a punctured portion. Then, the blood extracted by puncture with the puncture needle 3 comes into contact with the surfaces of the electrodes 20 to react to the chemical agent 30. As a result, a resistance value when a voltage is applied between the individual electrodes 20 changes in accordance with an amount or value of blood sugar in the blood. At this time, to provide the contact between the punctured portion and the electrodes 20, the electrodes 20 of the circuit board portion 2 are brought closer thereto such that the main body portion 15 and the lateral portions 18 of the guard portion 4 are located on the opposite side of the punctured portion with respect to the circuit board portion 2.

Then, as shown in FIG. 3(d), the upstream end portion of the circuit substrate for blood collection 1 in the puncture direction is inserted into a terminal input portion 32 of the blood-sugar-value measuring device 31. Consequently, the terminals 21 of the circuit substrate for blood collection 1 and terminals (not shown) of the terminal input portion 32 come into contact with each other. The blood-sugar-value measuring device 31 is a device for easily measuring a blood sugar value, and has the same structure as that of each of various commercially available known devices. In the blood-sugar-value measuring device 31, when the terminals 21 of the circuit substrate for blood collection 1 have come into contact with the terminals (not shown) of the terminal input portion 32, a predetermined voltage is applied, and the amount of glucose is measured based on the changed resistance value. The measured amount of glucose is displayed as a blood sugar value on an LED display portion 33.

The circuit substrate for blood collection 1 includes the circuit board portion 2 and the puncture needle 3 formed integrally therewith. Therefore, it is possible to extract an extremely small amount of blood by puncture with the puncture needle 3, and easily bring the extracted blood into contact with the electrodes 20 of the circuit board portion 2. Additionally, in the circuit substrate for blood collection 1, the electrodes 20, the terminals 21, and the wires 22 are provided integrally as the conductive pattern 13 in the circuit board portion 2. Therefore, it is possible to improve the accuracy of sensing glucose in the blood that comes in contact with the electrodes 20, and improve measurement accuracy. As a result, the circuit substrate for blood collection 1 allows accurate measurement of glucose in blood with a simple structure, and allows easy operation.

In addition, in the circuit substrate for blood collection 1, the guard portion 4 can guard the tip 5 of the puncture needle 3, and therefore prevent damage to the puncture needle 3. As a result, it is possible to reliably prevent damage to the puncture needle 3 prior to puncture, and ensure bleeding with the tip 5 of the puncture needle 3 upon puncture to allow sensing of a component of blood.
Moreover, in the circuit substrate for blood collection 1, it is possible to bring the guard portion 4 and the puncture needle 3 away from each other using the bend portions 19, and release the guarding by the guard portion 4. Therefore, it is possible to prevent damage to the puncture needle 3 prior to puncture, while it is possible to easily release the guarding by the guard portion 4 at the time of puncture, and reliably expose the puncture needle 3.

Specifically, by bending the bend portions 19, the guard portion 4 and the puncture needle 3 can be reliably brought away from each other, and the guarding by the guard portion 4 can be reliably released.
Note that, in the description given above, the guard portion 4 is formed only from the metal board 11. However, by, e.g., further laminating the insulating base layer 12 and/or the insulating cover layer 14 on the metal board 11 in accordance with a strength required of the guard portion 4, the guard portion 4 can be formed integrally therewith, though not shown.

In the description given above, the stopper portions 8 are formed in the circuit board portion 2. However, in the case where a medical expert or a patient skilled in puncture uses the circuit substrate for blood collection 1, it is possible to obtain the circuit substrate for blood collection 1 without forming the stopper portions 8. In this case, the metal board 11 and the insulating base layer 12 can be formed more easily, and cost can be reduced.
In the description given above, the circuit substrate for blood collection 1 with the bend portions 19 being bent is inserted into the blood-sugar-value measuring device 31. However, after the contact between the punctured portion and the electrodes 20 (see FIG. 3(c)), the bend portions 19 can also be, e.g., bent again to be restored into the original state shown in FIG. 3(a), and inserted into the blood-sugar-value measuring device 31, though not shown.

Also in the description given above, the bend portions 19 are bent such that the main body portion 15 and the lateral portions 18 remain in the circuit board portion 2. However, it is also possible to, e.g., cut the metal board 11 along the bend portions 19 to separate the main body portion 15 and the lateral portions 18 from the base portions 17, and remove the main body portion 15 and the lateral portions 18 from the circuit board portion 2, though not shown.
FIGS. 4 and 5 are illustrative views each showing an example of a method of using a circuit substrate for blood collection which is another embodiment of the circuit board for body fluid collection of the present invention, in which FIG. 4 shows an implementation in which guard-side groove portions are formed in bend portions, and FIG. 5 shows an implementation in which guard-side through holes are formed in bend portions. Note that, in each of the drawings shown below, the same members as described above are provided with the same reference numerals, and a description thereof is omitted.

In the description given above, the bend portions 19 are formed of the guard-side recessed portions 26. However, it is sufficient for the bend portions 19 to be provided as, e.g., fragile portions which allow the main body portion 15 and the lateral portions 18 to be bent with respect to the base portions 17. As shown in FIG. 4, the bend portions 19 can be formed of guard-side groove portions 27 in which the lower portion of the metal board 11 is recessed upwardly over the entire widthwise direction in the adjoining portions between the base portions 17 and the lateral portions 18.

In FIG. 4, each of the guard-side groove portions 27 is formed such that a cross section thereof along the puncture direction has a generally rectangular shape. The length of the guard-side groove portion 27 in the puncture direction is in a range of, e.g., 10 to 1000 m, or preferably 15 to 500 µm. The depth of the guard-side groove portion 27 is in a range of, e.g., 10 to 40 µm, or preferably 15 to 30 µm.
Alternatively, as shown in PIG. 5, it is also possible to form the bend portions 19 of guard-side through holes 28 extending through the metal board 11 in the thickness direction at widthwise midpoints in the adjoining portions between the lateral portions 18 and the base portions 17.

Each of the guard-side through holes 28 is formed in, e.g., a generally cylindrical shape. The inner diameter of the guard-side through hole 28 is in a range of, e.g., 100 to 4000 µm, or preferably 300 to 3000 µm. In FIG. 5, the guard-side through holes 28 are formed correspondingly to the individual lateral portions 18 in one-to-one relation. However, a plurality of the guard-side through holes 28 can be formed for each of the lateral portions 18, though not shown in FIG. 5. In this case, the plurality of guard-side through holes 28 are arranged in widthwise spaced-apart relation.

FIG. 6 shows a circuit substrate for blood collection which is yet another embodiment of the circuit board for body fluid collection of the present invention, (a) showing a plan view, and (b) showing a longitudinal cross-sectional view along the line B-B in (a). FIGS. 7 to 9 are illustrative views each showing an example of a method of using the circuit substrate for blood collection which is the yet another embodiment of the circuit board for body fluid collection of the present invention, of which FIG. 7 shows an implementation in which circuit-side recessed portions are formed in a bend portion, FIG. 8 shows an implementation in which a circuit-side groove portion is formed in a bend portion, and FIG. 9 shows an implementation in which circuit-side through holes are formed in a bend portion.

In the description given above, the bend portions 19 are provided in the guard portion 4, and the main body portion 15 is bent. However, as shown in FIGS. 6 to 9, it is also possible to, e.g., provide the bend portion 19 in the midway portion of the circuit board portion 2 in the puncture direction.
In FIG. 6, the bend portion 19 is formed in the midway portion of the circuit board portion 2 in the puncture direction. Specifically, the bend portion 19 is formed in the portion of the circuit board portion 2 where the wires 22 are formed, and formed in the same line as the downstream end edges of the base portions 17 in the puncture direction along the widthwise direction. In the both widthwise outer end portions of the bend portion 19, circuit-side recessed portions 36 are formed.

As shown in FIG. 6(b), the circuit-side recessed portions 36 are each formed of a substrate-side recessed portion 39 extending through the metal board 11 in the thickness direction, a base-side recessed portion 40 extending through the insulating base layer 12 in the thickness direction, and a cover-side recessed portion 41 extending through the insulating cover layer 14 in the thickness direction. Each of the substrate-side recessed portion 39, the base-side recessed portion 40, and the cover-side recessed portion 41 has the same plan view shape as that of each of the guard-side recessed portions 26 shown in FIG. 1 described above.

In the measurement of an amount of glucose in blood using the circuit substrate for blood collection 1, the guarding of the tip 5 of the puncture needle 3 by the guard portion 4 is released first, as shown by the arrow of FIG. 7. That is, the puncture needle 3 and the downstream end portion of the circuit board portion 2 in the puncture direction are bent along the bend portion 19 with respect to the upstream end portion of the circuit board portion 2 in the puncture direction. By the bending, as shown by the solid line of FIG. 7, the puncture needle 3 is rotationally moved with respect to the upstream end portion of the circuit board portion 2 in the puncture direction toward the top surface side (side with the insulating cover layer 14) of the circuit board portion 2 in the direction of rotational movement.

In this manner, the guarding of the tip 5 of the puncture needle 3 by the guard portion 4 is released.
In the circuit substrate for blood collection 1, by bending the bend portion 19, the guard portion 4 and the puncture needle 3 can be reliably brought away from each other to allow reliable release of the guarding by the guard portion 4.
ln the description given above, the bend portion 19 is provided in the circuit board portion 2. However, it is also possible to provide the bend portion 19 in, e.g., the puncture needle 3, specifically in the portion of the puncture needle 3 located upstream of the tip 5 thereof in the puncture direction, i.e., in the midway portion of the puncture needle 3 in the puncture direction or in the upstream end portion of the puncture needle 3 in the puncture direction, though not shown.

Also, in the description of FIGS. 6 and 7 given above, the bend portion 19 is formed of the circuit-side recessed portions 36. However, it is sufficient for the bend portion 19 to be formed as a fragile portion, similarly to the bend portions 19 of FIGS. 1, 4, and 5. For example, as shown in FIG. 8, the bend portion 19 can be formed of a circuit-side groove portion 37 which is the lower portion of the metal board 11 recessed upwardly over the entire widthwise direction at the upstream end edge of the circuit board portion 2 in the puncture direction. The circuit-side groove portion 37 is formed in the same shape as that of the guard-side groove portion 27 described above.

As shown in FIG. 9, the bend portion 19 can also be formed of a plurality of guard-side through holes 38 extending through the metal board 11 in the thickness direction thereof at widthwise midpoints in the downstream end portion of the circuit board portion 2 in the puncture direction. The guard-side through holes 38 are formed in the same shapes as those of the guard-side through holes 28. Note that, when viewed in plan view, the guard-side through holes 38 are disposed between the wires 22 so as not to overlap the positions where the wires 22 are formed.

FIG. 10 is an illustrative view showing an example of a method of using a circuit substrate for blood collection which is still another embodiment of the circuit board for body fluid collection of the present invention, and shows an implementation in which the circuit board portion also serves as the guard portion.
In the description given above, the circuit board portion 2 and the guard portion 4 are provided as distinct members in the circuit substrate for blood collection 1. However, it is also possible to, e.g., cause the guard portion 4 to serve also as the circuit board portion 2.

In FIG. 10, each of the circuit board portion 2 and the guard portion 4 includes the metal board 11, the insulating base layer 12, the conductive pattern 13, and the insulating cover layer 14.
In the guard portion 4, a terminal formation portion 34 protruding in a generally rectangular plan view shape from the main body portion 15 toward the downstream side in the puncture direction is provided upstream of the main body portion 15 in the puncture direction. In the terminal formation portion 34, the terminals 21 exposed from the insulating cover layer 14 are formed. In addition, the wires 22 are routed in the main body portion 15 and the support portion 16.

Thus, the guard portion 4 serves also as the circuit board portion 2.
In the circuit substrate for blood collection 1, the guard portion 4 serves also as the circuit board portion 2, and therefore it is possible to effectively use the space for the guard portion 4, and improve layout flexibility.
In the description given above, the circuit substrate for blood collection 1 is shown as an example of the circuit board for body fluid collection of the present invention. However, the circuit board for body fluid collection of the present invention is not limited to blood collection. A target object to be measured is not particularly limited as long as it is a fluid present in a living body. For example, an intracellular fluid or an extracellular fluid can be measured as the target object. Examples of the extracellular fluid that can be listed include a blood plasma, an interstital fluid, a lymph fluid, moistures in dense connective tissue, bone, and cartilage, and a transcellular fluid, apart from blood mentioned above.

### EXAMPLES

Hereinbelow, the present invention is described specifically by showing the examples. However, the present invention is by no means limited to the examples.

### EXAMPLE 1 (Production of Circuit Substrate for Blood Collection Shown in FIG. 1)

First, a metal board made of SUS430, and having a thickness of 50 µm and a width of 350 mm was prepared (see FIG. 2(a)).

Then, on the surface of the metal board, a varnish of a photosensitive polyimide resin precursor (photosensitive polyamic acid resin) was coated, and dried by heating to form a coating. The coating was then exposed to light, and developed to be formed into a pattern. Thereafter, the coating was heated in a nitrogen atmosphere to 400 °C to form an insulating base layer having a thickness of 10 µm in the foregoing pattern (see FIG. 2(b)).
Then, on the surface of the insulating base layer, a chromium thin film and a copper thin film were successively formed by sputtering to form a metal thin film. Thereafter, a dry film resist was laminated on the surface of the metal thin film, exposed to light, and developed to form a plating resist in a pattern. Then, a plated layer made of copper was formed on the surface of the metal thin film exposed from the plating resist by electrolytic copper plating using the metal thin film as a seed film to form a conductive pattern including electrodes, terminals, and wires (see FIG. 2(c)). Thereafter, the plating resist and the metal thin film on the portion where the plating resist was formed were removed by etching.

The thickness of the conductive pattern was 12 µm. The length of one side of each of the electrodes was 0. 3 mm. The length of one side of each of the terminals was 2 mm. The length of each of the wires was 25 mm.
Thereafter, on the surface of the insulating base layer, a varnish of a photosensitive polyimide resin precursor (photosensitive polyamic acid resin) was coated so as to cover the conductive pattern, and dried by heating to form a coating. The coating was then exposed to light, and developed to be formed into a pattern. Thereafter, the coating was heated in a nitrogen atmosphere to 400 °C to form an insulating cover layer having a thickness of 5 µm (see FIG. 2(d)). Note that the insulating cover layer was formed so as to expose the electrodes and the terminals, and cover the wires.

Then, on each of the exposed surfaces of the electrodes and the terminals, an electrolytic nickel plated layer (having a thickness of 0.5 µm) and an electrolytic gold plated layer (having a thickness of 2.5 µm) were successively formed.
Then, a dry film resist was laminated on the surface of the metal board, exposed to light, and developed to form an etching resist in a pattern. Then, the metal board exposed from the etching resist was etched by wet etching using a ferric chloride solution as an etchant, and trimmed into the foregoing pattern having a circuit substrate, a puncture needle, and a guard portion (see FIG. 2(e)). Note that, concurrently with the trimming of the metal board, guard-side recessed portions each cut into a semi-cylindrical shape and stopper portions were formed simultaneously.

The widthwise length of the circuit board portion was 0.3 mm, and the longitudinal length thereof was 5 mm. The distance from the tip of the puncture needle to the nearest electrode was 0.5 mm, and the angle of the tip of the puncture needle was 20°. The widthwise protruding length of each of the stopper portions was 0.5 mm, and the separation distance between the downstream end edge of the stopper portion in the puncture direction and the tip of the puncture needle was 1 mm.
In the guard portion, the length (L1 of a main body portion was 5.0 mm, the length (L2) of each of the base portions was 2 mm, the length (L3) of each lateral portion was 6 mm, the distance (L4) between the main body portion and the puncture needle in the puncture direction was 0.13 mm, and the widthwise distance (L5) between the lateral portion and the stopper portion was 0.8 mm. The width (W1) of each support portion and the width (W2) of the main body portion were each 0.5 mm. Note that the inner diameter of each of the guard-side recessed portions was 300 µm.

In this manner, a circuit substrate for blood collection was obtained. In the obtained circuit substrate for blood collection, a chemical agent containing a glucose oxidase and potassium ferricyanide solution was coated on one of the electrodes by an ink jet method (see FIG. 2(f)).

### Evaluation

By bending the lateral portions and the main body portion along the bend portions toward the metal board with respect to the base portions, the guarding by the guard portion was released (see FIG. 3(a)). This allowed the prevention of damage to the tip of the puncture needle prior to puncture.

Then, a fingertip was punctured with the tip of the puncture needle (see FIG. 3(b)), and the electrodes were brought closer into contact with a blood drop squeezed out therefrom (see FIG. 3(c)). As a result, by the blood, glucose was oxidized and ferricyanide ions reacted so that the circuit substrate for blood collection was inserted into a blood-sugar-value measuring device (see FIG. 3(d)), which allowed measurement of an amount of glucose. Note that, during the puncture, the stopper portions came in contact with skin to be able to prevent the puncture needle from deeply piercing the skin.

### EXAMPLE 2 (Production of Circuit Substrate for Blood Collection Shown in FIG. 6)

A circuit substrate for blood collection was obtained (see FIG. 6) in the same manner as in Example I except that circuit-side recessed portions were formed instead of the guard-side recessed portions in the trimming of a metal board. Each of the circuit-side recessed portions was cut in a semi-cylindrical shape, and the inner diameter thereof was 1000 µm.

### Evaluation

By bending the puncture needle and the downstream end portion of the circuit board portion in the puncture direction along the bend portion toward the insulating cover layer with respect to the upstream end portion of the circuit board portion in the puncture direction, the guarding by the guard portion was released (see FIG. 7(a)). This allowed the prevention of damage to the tip of the puncture needle prior to puncture.

Then, a fingertip was punctured with the tip of the puncture needle (see FIG. 3(b)), and the electrodes were brought closer into contact with a blood drop squeezed out therefrom (see FIG. 3(c)). As a result, glucose was oxidized by the blood, and ferricyanide ions reacted so that the circuit substrate for blood collection was inserted into a blood-sugar-value measuring device (see FIG. 3(d)), which allowed measurement of an amount of glucose. Note that, during the puncture, the stopper portions came in contact with skin to be able to prevent the puncture needle from deeply piercing the skin.

While the above description has been given as the illustrative embodiments of the present invention, such is for illustrative purpose only and it is not to be construed imitative. Modification and variation of the present invention which will be obvious to those skilled in the art is to be covered by the following claims.

### Industrial Applicability

A circuit board for body fluid collection of the present invention is connected to a device for measuring a component of a body fluid such as blood, and used to measure a component of the body fluid such as an amount of glucose in blood.

## Claims

1. A circuit board for body fluid collection comprising:
a circuit board portion including an insulating layer, and a conductive pattern supported on the insulating layer and integrally including an electrode, a terminal for connecting to a device for measuring a component of a body fluid, and a wire electrically connecting the electrode and the terminal;
a puncture needle integrally formed with the circuit board portion, and protruding from the circuit board portion in order to collect the body fluid by puncture; and
a guard portion disposed downstream of the puncture needle in a puncture direction in opposing relation thereto in order to guard a tip of the puncture needle.

2. The circuit board for body fluid collection according to Claim 1, further comprising:
a releasing portion for releasing the guarding of the tip of the puncture needle by the guard portion, by bringing the guard portion and the puncture needle away from each other.

3. The circuit board for body fluid collection according to Claim 2, wherein
the guard portion is supported by the circuit board portion, and
the releasing portion is bendably provided in a portion of the guard portion located upstream of the tip of the puncture needle in the puncture direction.

4. The circuit board for body fluid collection according to Claim 2, wherein the releasing portion is bendably provided in a portion of the puncture needle or the circuit board portion located upstream of the tip of the puncture needle in the puncture direction.
